(19) Europäisches Patentamt
European Patent Office
Office européen des brevets

(11) Publication number: **0 175 644 A2**

(12) # EUROPEAN PATENT APPLICATION

(21) Application number: 85810399.7

(51) Int. Cl.⁴: **A 61 K 37/02**

(22) Date of filing: 03.09.85

(30) Priority: 17.09.84 GB 8423431

(43) Date of publication of application: 26.03.86 Bulletin 86/13

(84) Designated Contracting States: AT BE CH DE FR GB IT LI LU NL SE

(71) Applicant: SANDOZ AG, Lichtstrasse 35, CH-4002 Basel (CH)

(84) Designated Contracting States: BE CH FR GB IT LI LU NL SE

(71) Applicant: SANDOZ-PATENT-GMBH, Humboldtstrasse 3, D-7850 Lörrach (DE)

(84) Designated Contracting States: DE

(71) Applicant: SANDOZ-ERFINDUNGEN Verwaltungsgesellschaft m.b.H., Brunner Strasse 59, A-1235 Wien (AT)

(84) Designated Contracting States: AT

(72) Inventor: Doepfner, Wolfgang, Oberwilerstrasse 15, CH-4054 Basel (CH)
Inventor: Flückiger, Edward, Im Marteli 9, CH-4102 Binningen (CH)

(54) **New pharmaceutical use of somatostatin analogues.**

(57) Use of the compound of formula

H-(D)Phe-Cys-Phe-(D)Trp-Lys-Thr-Cys-Thr-ol

and analogues thereof for the treatment of diseases involving morbid proliferation or keratinisation of epidermal cells, in particular psoriasis.

0175644



Case 100-6446

# NEW PHARMACEUTICAL USE OF SOMATOSTATIN ANALOGUES

The present invention relates to a new use, in particular a new pharmaceutical use of somatostatin analogues.

The somatostatin analogues to which the present invention relates are the compounds of formula I

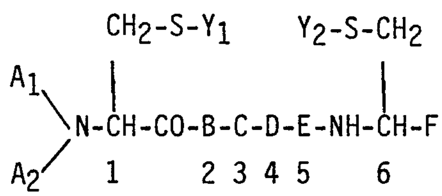

(I)

wherein

$A_1$ is 1) $C_{1-12}$alkyl or $C_{7-10}$phenylalkyl;

2) a group of formula RCO-, wherein

2.1) R is H, $C_{1-11}$alkyl, phenyl or $C_{7-10}$phenylalkyl, or

2.2) RCO- is a) an L- or D-phenylalanine residue optionally ring-substituted by F, Cl, Br, $NO_2$, $NH_2$, OH, $C_{1-3}$alkyl and/or $C_{1-3}$alkoxy,

b) the residue of a natural α-amino acid other than defined under a) above or of a corresponding D-amino acid, or

c) a dipeptide residue in which the individual amino acid residues are the same or different and are selected from those defined under a) and/or b) above,

the $\alpha$-amino group of amino acid residues a) and b) and the N-terminal amino group of dipeptide residues c) being optionally mono- or di-$C_{1-12}$alkylated; or

3) A group of formula 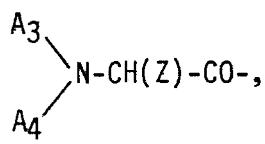 wherein

$A_3$ is a group of formula

a) $R^{I}CO-$ wherein $R^{I}$ is an aliphatic, cycloaliphatic, aromatic or heterocyclic group;

b) $R^{II}SO_2-$ wherein $R^{II}$ is $C_{1-10}$alkyl, phenyl or $C_{7-10}$-(phenylalkyl);

c) $R^{III}O-CO-$ wherein $R^{III}$ is $C_{1-10}$alkyl or $C_{7-10}$-(phenylalkyl); or

d) 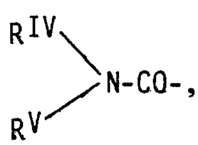 , 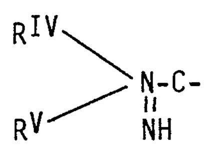 or 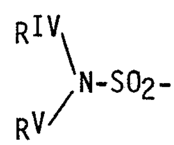

wherein $R^{IV}$ is hydrogen, $C_{1-10}$alkyl, phenyl or $C_{7-10}$(phenylalkyl) and

$R^{V}$ is hydrogen or $C_{1-10}$alkyl,

$A_4$ is hydrogen or $C_{1-3}$alkyl, and

>N-CH(Z)-CO- has the meaning a) or b) given above for RCO-,

$A_2$ is hydrogen or, when $A_1$ has the meaning given under 1) above, also $C_{1-12}$alkyl or $C_{7-10}$phenylalkyl.

B is -Phe- optionally ring-substituted by F, Cl, Br, $NO_2$, $NH_2$, OH, $C_{1-3}$alkyl and/or $C_{1-3}$alkoxy,

C is -Trp- or (D)-Trp- optionally α-N-methylated and optionally benzene-ring-substituted by F, Cl, Br, $NO_2$, $NH_2$, OH, $C_{1-3}$-alkyl and/or $C_{1-3}$alkoxy,

D is -Lys- optionally α-N-methylated and optionally ε-N-$C_{1-3}$-alkylated,

E is the residue of a natural α-amino acid or of a corresponding (D)-amino acid, said residue being optionally α-N-methylated,

F is a group of formula $-COOR_1$, $-CH_2OR_2$, 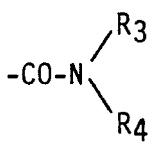 or

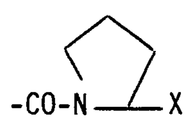

wherein $R_1$ is hydrogen or $C_{1-3}$alkyl,

$R_2$ is hydrogen or the residue of a physiologically acceptable, physiologically hydrolysable ester,

$R_3$ is hydrogen, $C_{1-3}$alkyl, phenyl or $C_{7-10}$phenylalkyl,

$R_4$ is hydrogen, $C_{1-3}$alkyl or, when $R_3$ is hydrogen or methyl, also a group of formula $-CH(R_5)-X$,

$R_5$ is hydrogen, $-(CH_2)_2-OH$ or $-(CH_2)_3-OH$, or represents the substituent attaching to the α-carbon atom of a natural α-amino acid and

X is a group of formula $-COOR_1$, $-CH_2OR_2$ or

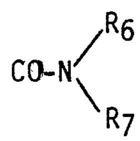 wherein

$R_1$ and $R_2$ have the meanings given above,
$R_6$ is hydrogen or $C_{1-3}$alkyl and
$R_7$ is hydrogen, $C_{1-3}$alkyl, phenyl or $C_{7-10}$phenylalkyl,

the group $-CH(R_5)-X$ having the (D)- or(L)-configuration, and

$Y_1$ and $Y_2$ are each hydrogen or together represent a direct bond,

whereby the residues in the 1- and 6-position each independently have the (L)- or (D)-configuration, and with the proviso that (L)- and/or (D)-cysteine residues are present at the 1- and 6-positions only.

The said somatostatin analogues of formula I are disclosed together with methods for their production and their use in the treatment of disorders with an aetiology comprising or associated with excess GH-secretion, e.g. in the treatment of diabetes mellitus, angiopathy and acromegaly, as well as in the treatment of gastro-intestinal disorders, e.g. in the treatment of gastric ulcer, gastro-intestinal bleeding and acute pancreatitis, inter al. in European Patent Publication No. 0 029 579 B1 [≡ US Patent No. 4,395,403: compounds of formula I in which $A_1$ has the meanings given under 1) and 2) above] and UK Patent Specification No. 2 095 264 [≡ US Patent No. 4,435,385: compounds of formula I in which $A_1$ has the meanings given under 3) above].

Preferred sub-groups for compounds of formula I are as set-forth and defined in the aforementioned European Patent Publication, UK Patent Specification and US Patents.

The most preferred somatostatin analogue for use in accordance with the present invention is the compound of formula Ia

H-(D)Phe-Cys-Phe-(D)Trp-Lys-Thr-Cys-Thr-ol (Ia)

in which -Thr-ol represents the L-threoninol residue of formula

$$\begin{array}{c} CH_3 \\ | \\ CH(OH) \\ | \\ -NH-CH-CH_2OH \\ (L) \end{array}$$

hereinafter referred to as SMS, the synthesis of which is specifically described in the aforementioned European Patent Publication.

Further preferred somatostatin analogue for use in accordance with the present invention are the compounds of formula Ib and Ic

$CH_3$-CO(D)Phe-Cys-Phe-(D)Trp-Lys-Thr-Cys-Thr-ol (Ib)

$CH_3-(CH_2)_8$-CO-(D)Phe-Cys-Phe-(D)Trp-Lys-Thr-Cys-Thr-ol (Ic)

in which -Thr-ol has the meaning given above, hereinafter referred to as COMPOUND II and COMPOUND III respectively, the synthesis of which is specifically disclosed in the aforementioned UK Patent Specification.

The said somatostatin analogues exist, and may be used in accordance with the present invention, in free form or in pharmaceutically acceptable salt or complex form. Such salt and complex forms are also known from the aforementioned patent references and include in the case of SMS in particular the acetate. In general such salt and complex forms exhibit the same or similar order of activity and tolerability as the free forms and references throughout the present specification and claims to somatostatin analogues of formula I as well as the compounds SMS, COMPOUND II and COMPOUND III, are to be understood as including both free, and pharmaceutically acceptable acid addition salt and complex forms unless otherwise specified.

In accordance with the present invention it has now surprisingly been found that the somatostatin analogues of formula I, in particular SMS, COMPOUND II and COMPOUND III, are effective in inhibiting the proliferation and/or keratinisation of epidermal cells and are useful in the treatment of dermatological diseases involving morbid proliferation and/or keratinisation of epidermal cells, in particular in the treatment of psoriasis.

Effectiveness of the said compounds in the treatment of e.g. psoriasis may be demonstrated in clinical trials, conducted e.g. as follows:

The trial is conducted employing adult ♀ and ♂ subjects exhibiting chronic severe psoriasis involving 20 % or more of the body

surface as estimated by the rule of nines, and whose disease condition is either stable or progressive on current therapy. All systemic, topical or phototherapy specifically directed at skin disease is stopped for at least 2 weeks prior to commencement of the trial. Only bland emollients, 2 % salicyclic acid in olive oil, coal tar shampoos, and/or 1 % hydrocortisone cream or ointment and medication for arthritis or other concomitant disease to psoriasis is continued during this period.

After the 2-week "washout" period, subjects are admitted to the trial after an overnight fast of 8 - 10 hours. On admission, the following laboratory tests are performed: complete blood-count, serum electrolytes, glucose, calcium, phosphorous, uric acid, ALT-AST-LDH (alanine amino transferase/aspartate aminotransferase/lactate dehydrogenase), plasma epidermal growth factor, growth hormone, gut peptide profile (gastrin, somatostatin, VIP (vasoactive intestinal peptide), insulin, glucagon, PP (pancreatic polypeptide), neurotensin, substance P, motilin, prolactin, gastrin releasing peptide, GIP (gastrin inhibiting peptide) and calcitonin) and urine EGF. Clinical extent of psoriasis is estimated by rule of nines, and lesions are graded for redness, thickness and scaliness using a 0-4 scale as described by Kragballe et al. Arch. dermatol. 119, 548-552 (1983). Clinical photographs are taken of selected lesions. A 6 mm punch skin biopsy is performed using 1% lidocaine infiltration anaesthesia.

During the course of the trial subjects receive:

i) 0.1 mg SMS in the form of its acetate/day for the first 3 days, administered s.c. in 2 doses of 0.05 mg each, once in the morning and once in the evening

ii) 0.2 mg SMS in the form of its acetate/day for the remaining

25 days, administered s.c. in 2 doses of 0.1 mg each, (mornings and evenings).

No other therapy is allowed during the trial period, except as already employed during the "washout" period. All testing carried out at entry into the trial (fasting blood, urine, estimation of clinical extent of psoriasis, clinical photography, punch skin biopsy) is repeated during the course of the trial on days 7, 14, 21 and 28.

Subjects taking part in the trial and receiving SMS in the indicated dose regimen exhibit marked improvement in psoriatic condition as evidenced by progressive reduction in clinical extent of psoriasis, in particular in the extent of psoriatic lesion, as well as by marked reduction in grading for lesion condition. In addition results from sequential punch skin biopsies, indicate marked histological change in relation to lesion condition, including reduction of mitotic index, reduction of inflammatory infiltrate and noticeable improvement in vasculature.

Efficacy of SMS therapy may also be proven on repetition of the trial in double blind, cross-over format employing two groups of patients, one group receiving SMS in accordance with the above indicated regimen and the other receiving placebo injections only, both groups being matched for extent of lesions.

Subjects receiving SMS show marked improvement with respect to parameters specifically listed above as compared with subjects receiving placebo only.

Similar results may be obtained employing other compounds of formula I in particular COMPOUND II and COMPOUND III.

As an alternative to s.c. administration, the somatostatin analogues may also be applied topically, e.g. at the site of psoriatic lesion, or by other non-parenteral route.

In accordance with the foregoing, the present invention provides a method for the treatment of dermatological disease involving morbid proliferation or keratinisation of epidermal cells, e.g. for the treatment of psoriasis, in a subject in need of such treatment, which method comprises administering to said subject an effective amount of a somatostatin analogue of formula I as hereinbefore defined, in particular of the compound SMS, COMPOUND II or COMPOUND III.

For this use, the dosage will, of course, vary depending on the mode of administration, the particular somatostatin analogue used and the treatment desired.

In general, for SMS, satisfactory results are obtained on administration at a daily dosage in the range of from about 0.01 to about 2.5, preferably about 0.05 to about 0.5, especially about 0.1 to about 0.2 mg/day conveniently administered in divided dosages 2 to 4 times a day or in sustained release form. Dosage forms suitable for parenteral (e.g. s.c., i.v. or i.m.) administration accordingly comprise from about 0.0025 to about 1.25, preferably from about 0.0125 to about 0.25 or 0.5, especially from about 0.025 to about 0.1 mg SMS admixed with an appropriate diluent or carrier therefor.

Dosages for other somatostatin analogues of formula I, in particular COMPOUND II and COMPOUND III will be of corresponding order.

Where appropriate, initial treatment may subsequently be followed by a second course of therapy with a similar or, where appropriate, reduced dosage regimen.

The somatostatin analogues may be administered in the form of a pharmaceutical composition comprising the somatostatin analogue in free form or in pharmaceutically acceptable salt or complex form in association with a pharmaceutically acceptable carrier or diluent. Suitably they are put up in a form suitable for injection, e.g. for parenteral administration.

Alternatively they may be put up in the form of creams or ointments or solutions for topical application or in tablet or capsule form for systemic application.

Suitable forms for injection may be prepared in accordance with standard techniques, e.g. in accordance with the following example:

EXAMPLE

The following ingredients are employed to prepare injection solutions comprising 0.025, 0.05, 0.1 and 0.5 mg active ingredient (SMS)/ml).

| | | SOLUTION CONCENTRATION | | | |
|---|---|---|---|---|---|
| | INGREDIENT | 0.025 mg/ml | 0.05 mg/ml | 0.1 mg/ml | 0.5 mg/ml |
| 1. | SMS (as the acetate) | 0.05 mg | 0.05 mg | 0.1 mg | 0.5 mg |
| 2. | Sodium acetate ($3H_2O$) | 3.60 mg | 3.60 mg | 3.60 mg | 3.60 mg |
| 3. | Acetic acid (99 - 100 %) | 6.40 mg | 6.40 mg | 6.40 mg | 6.40 mg |
| 4. | NaCl | 7.00 mg | 7.00 mg | 7.00 mg | 7.00 mg |
| 5. | $H_2O$ (injection grade) added to an end volume of.... | 2.0 ml | 1.0 ml | 1.0 ml | 1.0 ml |

The injection solutions are prepared in accordance with standard techniques, filtered to sterilise and filled into ampoules under sterile conditions with $CO_2$-gassing. The ampoules are sealed and further sterilised by heating at 121°C for 5 minutes.

In accordance with the foregoing the present invention additionally provides:

I. A somatostatin analogue of formula I as hereinbefore defined, in particular SMS, COMPOUND II or COMPOUND III, for use in a method of treatment as hereinbefore defined; as well as

II. A somatostatin analogue of formula I as hereinbefore defined, in particular SMS, COMPOUND II or COMPOUND III, for use in the preparation of a pharmaceutical composition for use in a method of treatment as hereinbefore defined.

CLAIMS

1. A method for the treatment of dermatological disease involving morbid proliferation keratinisation of epidermal cells in a subject in need of such treatment, which method comprises administering to said subject an effective amount of a somatostatin analogue of formula I

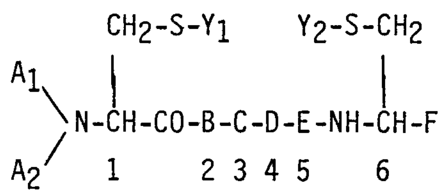

(I)

wherein

$A_1$ is 1) $C_{1-12}$alkyl or $C_{7-10}$phenylalkyl;

2) a group of formula RCO-, wherein

2.1) R is H, $C_{1-11}$alkyl, phenyl or $C_{7-10}$phenylalkyl, or

2.2) RCO- is a) an L- or D-phenylalanine residue optionally ring-substituted by F, Cl, Br, $NO_2$, $NH_2$, OH, $C_{1-3}$alkyl and/or $C_{1-3}$alkoxy,

b) the residue of a natural α-amino acid other than defined under a) above or of a corresponding D-amino acid, or

c) a dipeptide residue in which the individual aminoacid residues are the same or different and are selected from those defined under a) and/or b) above,

the $\alpha$-amino group of amino acid residues a) and b) and the N-terminal amino group of dipeptide residues c) being optionally mono- or di-$C_{-12}$alkylated; or

3) A group of formula 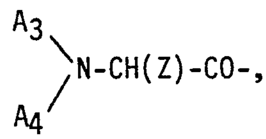, wherein

$A_3$ is a group of formula

a) $R^{I}CO-$ wherein $R^{I}$ is an aliphatic, cycloaliphatic, aromatic or heterocyclic group;

b) $R^{II}SO_2-$ wherein $R^{II}$ is $C_{1-10}$alkyl, phenyl or $C_{7-10}$-(phenylalkyl);

c) $R^{III}O-CO-$ wherein $R^{III}$ is $C_{1-10}$alkyl or $C_{7-10}$-(phenylalkyl); or

d) 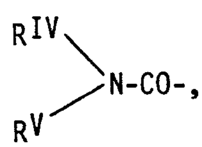 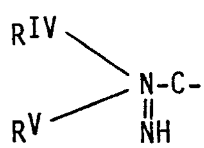 or 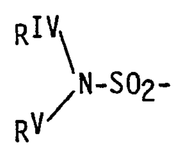

wherein $R^{IV}$ is hydrogen, $C_{1-10}$alkyl, phenyl or $C_{7-10}$(phenylalkyl) and

$R^{V}$ is hydrogen or $C_{1-10}$alkyl,

$A_4$ is hydrogen or $C_{1-3}$alkyl, and

$\geq$N-CH(Z)-CO- has the meaning a) or b) given above for RCO-,

$A_2$ is hydrogen or, when $A_1$ has the meaning given under 1) above, also $C_{1-12}$alkyl or $C_{7-10}$phenylalkyl.

B is -Phe- optionally ring-substituted by F, Cl, Br, $NO_2$, $NH_2$, OH, $C_{1-3}$alkyl and/or $C_{1-3}$alkoxy,

C is -Trp- or (D)-Trp- optionally $\alpha$-N-methylated and optionally benzene-ring-substituted by F, Cl, Br, $NO_2$, $NH_2$, OH, $C_{1-3}$-alkyl and/or $C_{1-3}$alkoxy,

D is -Lys- optionally $\alpha$-N-methylated and optionally $\varepsilon$-N-$C_{1-3}$-alkylated,

E is the residue of a natural $\alpha$-amino acid or of a corresponding (D)-amino acid, said residue being optionally $\alpha$-N-methylated,

F is a group of formula $-COOR_1$, $-CH_2OR_2$, 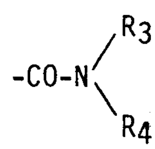 or

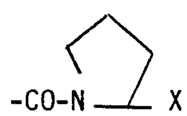

wherein $R_1$ is hydrogen or $C_{1-3}$alkyl,

$R_2$ is hydrogen or the residue of a physiologically acceptable, physiologically hydrolysable ester,

$R_3$ is hydrogen, $C_{1-3}$alkyl, phenyl or $C_{7-10}$phenyl-alkyl,

$R_4$ is hydrogen, $C_{1-3}$alkyl or, when $R_3$ is hydrogen or methyl, also a group of formula $-CH(R_5)-X$,

$R_5$ is hydrogen, $-(CH_2)_2-OH$ or $-(CH_2)_3-OH$, or re-presents the substituent attaching to the $\alpha$-carbon atom of a natural $\alpha$-amino acid and

X is a group of formula $-COOR_1$, $-CH_2OR_2$ or

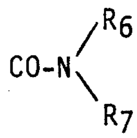 wherein

$R_1$ and $R_2$ have the meanings given above,
$R_6$ is hydrogen or $C_{1-3}$alkyl and
$R_7$ is hydrogen, $C_{1-3}$alkyl, phenyl or $C_{7-10}$phenylalkyl,

the group $-CH(R_5)-X$ having the (D)- or(L)-configuration, and

$Y_1$ and $Y_2$ are each hydrogen or together represent a direct bond,

whereby the residues in the 1- and 6-position each independently have the (L)- or (D)-configuration, and with the proviso that (L)- and/or (D)-cysteine residues are present at the 1- and 6-positions only.

2. A method according to claim 1 for the treatment of psoriasis.

3. The use of a somatostatin analogue of formula I as defined in claim 1 in a method of treatment as defined in claim 1 or 2.

4. The use of a somatostatin analogue of formula I as defined in claim 1 for the preparation of a pharmaceutical composition for use in a method of treatment as defined in claim 1 or 2.

5. A method according to claim 1 or 2, or use according to claim 3 or 4, wherein the somatostatin analogue is the compound of formula Ia

H-(D)Phe-Cys-Phe-(D)Trp-Lys-Thr-Cys-Thr-ol (Ia)

6. A method according to claim 1 or 2 or use according to claim 3 or 4 wherein the somatostatin analogue is the compound of formula Ib

$CH_3$-CO-(D)Phe-Cys-Phe-(D)Trp-Lys-Thr-Cys-Thr-ol (Ib)

7. A method according to claim 1 or 2 or use according to claim 3 or 4 wherein the somatostatin analogue is the compound of formula Ic

$CH_3-(CH_2)_8$-CO-(D)Phe-Cys-Phe-(D)Trp-Lys-Thr-Cys-Thr-ol (Ic)